(19) Europäisches Patentamt
European Patent Office
Office européen des brevets

(11) Veröffentlichungsnummer: **0 082 987**
**B1**

# (12) EUROPÄISCHE PATENTSCHRIFT

(45) Veröffentlichungstag der Patentschrift: **11.06.86**

(51) Int. Cl.[4]: **C 07 D 251/34**

(21) Anmeldenummer: **82111180.4**

(22) Anmeldetag: **02.12.82**

(54) **Neue Isocyanato-Isocyanurate sowie ein Verfahren zu deren Herstellung.**

(30) Priorität: **30.12.81 DE 3151855**

(43) Veröffentlichungstag der Anmeldung: **06.07.83 Patentblatt 83/27**

(45) Bekanntmachung des Hinweises auf die Patenterteilung: **11.06.86 Patentblatt 86/24**

(84) Benannte Vertragsstaaten: **AT BE CH DE FR GB IT LI LU NL SE**

(56) Entgegenhaltungen:
**EP - A - 0 013 880**
**DE - A - 2 325 826**

**Die Akte enthält technische Angaben, die nach dem Eingang der Anmeldung eingereicht wurden und die nicht in dieser Patentschrift enthalten sind.**

(73) Patentinhaber: **HÜLS AKTIENGESELLSCHAFT, Patentabteilung / PB 15 - Postfach 13 20, D-4370 Marl 1 (DE)**

(72) Erfinder: **Disteldorf, Josef, Dr., Am Sengenhoff 2a, D-4690 Herne 1 (DE)**
Erfinder: **Hübel, Werner, Dr., Birnenbruchstrasse 34, D-4690 Herne 1 (DE)**
Erfinder: **Wolf, Elmar, Dr., Stauffenbergstrasse 7, D-4350 Recklinghausen (DE)**

(74) Vertreter: **Stell, Hanna, Dipl.-Chem., RSP PATENTE - PB 15 Postfach 1320, D-4370 Marl 1 (DE)**

**EP 0 082 987 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Gegenstand der vorliegenden Erfindung sind Isocyanatoisocyanurate der Formel

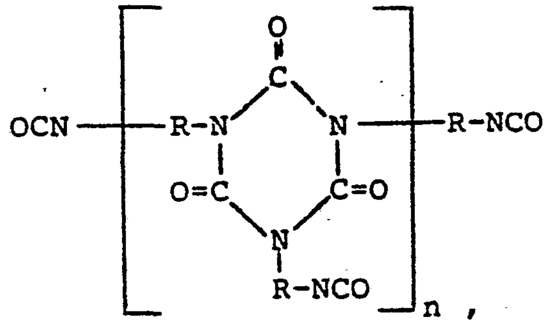

in der die einzelnen Reste R gleich oder verschieden sind und Kohlenwasserstoffreste der Formeln

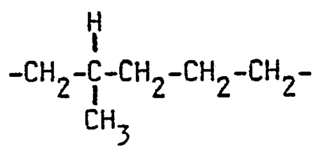 oder 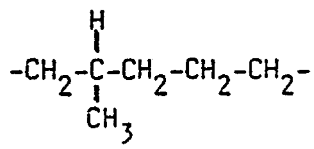 und 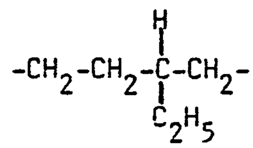.

bedeuten und n eine ganze oder gebrochene Zahl von 1 - 5 bedeutet.

Gegenstand der vorliegenden Erfindung ist auch ein Verfahren zur Herstellung von neuen Verbindungen durch partielle Trimerisierung von aliphatischen Diisocyanaten, dadurch gekennzeichnet, daß man als aliphatische Diisocyanate ein im wesentlichen aus einem Diisocyanat der Formel

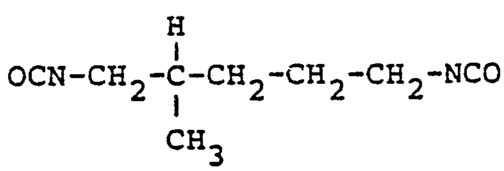

und gegebenenfalls einem Diisocyanat der Formel

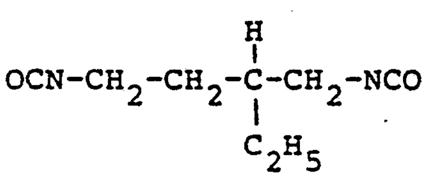

bestehendes Diisocyanat bzw. Diisocyanat-Gemisch verwendet, wobei der Mindestgehalt an 2-Ethyl-1,4-diisocyanatobutan 1 Gew.-% und die Menge an Katalysator 0,2-0,02 Gew.-% beträgt und der Temperaturbereich bei 40 - 140 °C liegt.

Die Trimerisierung von Hexamethylendiisocyanat (HDI) ist seit langem bekannt und wird in einer Vielzahl von Patentschriften mit verschiedenen Katalysatoren beschrieben (vgl. z.B. DE-ASsen 10 13 869, 12 03 792, 22 26 191; DE-OS 26 16 415; GB-PSsen 952 931, 966 338; US-PSsen 3 211 703, 3 330 828).

Keines dieser beschriebenen Verfahren gestattet auf technisch einfache Weise die problemlose Herstellung von praktisch farblosen, niedrigviskosen, lösungsmittelfreien Isocyanuratgruppen aufweisenden Polyisocyanaten auf HDI-Basis.

Erst in der DE-OS 28 39 133 wird die technische Herstellung des Isocyanato-isocyanurats auf HDI-Basis

aufgezeigt. Die nach diesem Verfahren erhaltenen Produkte weisen bei 25°C eine unter 10.000 mPas liegende Viskosität und einen NCO-Gehalt von 18 - 24, insbesondere von 20 - 23 Gew.%, auf und stellen wertvolle Ausgangsmaterialien zur Herstellung von lichtechten Polyurethan-Lacken dar.

Sie haben allerdings den Nachteil, daß sie mit den in PUR-Lacken im allgemeinen eingesetzten Polyhydroxylverbindungen nur beschränkt verträglich sind und insbesondere in den oft zur Anwendung gelangenden unpolaren Lösungsmitteln, wie z.B. Testbenzin, nur beschränkt löslich sind. Ein weiterer Nachteil dieser Isocyanatoisocyanurate des HDI ist ihre nur mäßige Thermo- wie Oxidationsbeständigkeit die vor allem beim Überbrennen der auf Basis dieser Verbindungen hergestellten Lackfilme offenkundig wird.

Des weiteren sind Isocyanato-isocyanurate gemäß EP-PS 13 880 auf Basis 5-Methyl-1,9-diisocyanato-nonan und 2,4-Dimethyl-1,8-diisocyanato-octan bekannt.

Es war daher die Aufgabe der vorliegenden Erfindung, neue Polyisocyanato-isocyanurate zur Verfügung zu stellen, die die bekannten Vorteile dieser Verbindungsklasse, wie hoher NCO-Gehalt, niedriger Dampfdruck, gute Lichtechtheit, lösungsmittelfrei verarbeitbar in sich vereinigen, ohne mit den genannten Nachteilen der Verbindungen des Standes der Technik (DE-OS 28 39 133) behaftet zu sein.

Diese Aufgabe konnte mit der Bereitstellung der eingangs erwähnten neuen Isocyanato-isocyanurate bzw. mit dem erfindungsgemäßen Verfahren zu ihrer Herstellung gelöst werden.

Ausgangsmaterialien für das erfindungsgemäße Verfahren sind Diisocyanat-Gemische, die im wesentlichen aus einem Diisocyanat der Formel

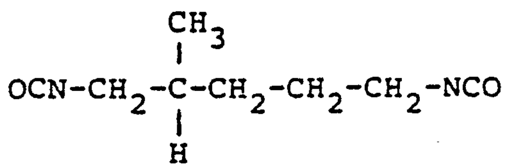

und einem Diisocyanat der Formel

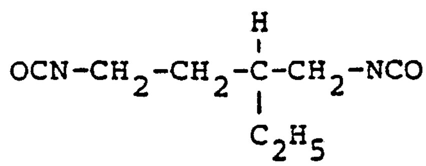

bestehen. Im allgemeinen weisen die beim erfindungsgemäßen Verfahren eingesetzten Diisocyanat-Gemische folgende Zusammensetzungen auf:

88 - 99 Gew.% 2-Methyl 1,5-diisocyanatopentan (MPDI)

12 - 1 Gew.% 2-Ethyl-1,4-diisocyanatobutan (EBDI)

Reines Diisocyanat der erstgenannten Formel kann anstelle der Gemische ebenfalls eingesetzt werden.

Bevorzugtes Ausgangsmaterial ist ein entsprechendes Diisocyanat-Gemisch aus ca. 88 - 95 Gew.% 2-Methyl-1,5-diisocyanatopentan und 12 - 5 Gew.% 2-Ethyl-1,4-diisocyanatobutan.

Die Herstellung der Diisocyanate bzw. Diisocyanat-Gemische erfolgt in an sich bekannter Weise durch Phosgenierung der entsprechenden Diamine (vgl. z.B. US-PS 3 631 198). Diese werden durch katalytische Hydrierung entsprechender Dinitrile erhalten, die beispielsweise als Nebenprodukt bei der Adipodinitrilherstellung durch Umsetzung von Butadien mit HCN oder bei der Dimerisierung von Acrylnitril anfallen.

Als Katalysatoren kommen für das erfindungsgemäße Verfahren im Prinzip alle an sich bekannten Trimerisierungskatalysatoren in Betracht. So lassen sich die gerade erwähnten Diisocyanate mit Na-Benzoat in Dimethylformamid (DMF) (Chem.Abstr. 60, 8332 (1963)) oder mit Na-Phenolaten in n-Butylacetat (GB-PS 1 386 399) gut trimerisieren. jedoch müssen die eben genannten Katalysatoren vom Reaktionsprodukt abgetrennt werden. Dieser Nachteil wird in der DE-OS 28 21 109 überwunden, indem man als Katalysator ein Mg- bzw. Ca-Salz der Monohydroxyethylphthalsäure einsetzt, das in das Reaktionsprodukt eingebaut wird. Für die Herstellung der erfindungsgemäßen Isocyanato-isocyanurate sind vor allem solche Katalysatoren geeignet, die nach beendeter Trimerisierung sich entweder leicht entfernen lassen oder bereits während der Reaktion vollständig desaktiviert werden. Als Beispiele für derartige Trimerisierungskatalysatoren seien genannt Aziridin (-derivate) in Kombination mit einem tertiären Amin (DE-AS 12 03 792, DE-OS 23 25 826); weiterhin die Kombination aus Alkylenoxid und N,N'-Endoethylenpiperazin (DE-OS 25 44 684) sowie quarternäre Ammoniumhydroxide (GB-PS 837 120).

Sehr gut geeignet erwies sich die Verwendung quarternärer Ammoniumsalze organischer Säuren gemäß DE-OS 29 16 201:

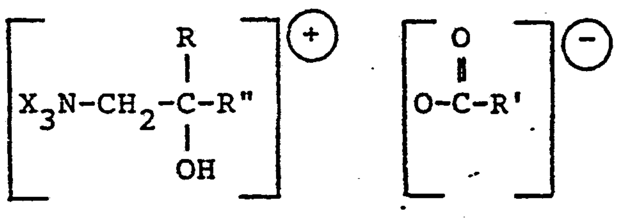

in der X gleiche oder verschiedene aliphatische, cycloaliphatische, araliphatische oder heterocyclische Kohlenwasserstoffreste eines quarternären Ammonium-Stickstoffs sind oder 2 X mit dem quarternären Stickstoff einen gegebenenfalls ein oder mehr Heteroatome enthaltenden Ring bzw. 3 X über ein gemeinsames Heteroatom mit dem quarternären Stickstoff einen Ring bilden, R ein Alkyl-, Cycloalkyl- oder Aralkylrest, sowie R und R″ gemeinsam ein $C_1$-$C_{12}$-Alkylenrest sein kann und R′ Wasserstoff, eine Hydroxylgruppe und ein gegebenenfalls hydroxylgruppenhaltiger $C_1$-$C_{12}$-Alkylrest und R″ = R oder Wasserstoff bedeuten können.

Der Katalysator muß in Mengen von 0,2 - 0,02 Gew.%, vorzugsweise 0,08 - 0,04 Gew.%, zu den trimerisierenden Diisocyanatgemischen zugesetzt werden; die Trimerisierung wird in einem Temperaturbereich von 40 - 140° C, vorzugsweise 60 - 90° C, durchgeführt. Die Trimerisierung ist bei diesen Temperaturen innerhalb von 1 - 60 Minuten beendet.

Bei der chargenweisen Trimerisierung des Diisocyanatgemisches (MPDI + EBDI) nach dem erfindungsgemäßen Verfahren wurden das Diisocyanatgemisch und 0,02 - 0,2 Gew.-Teile des beschriebenen Katalysators unter guter Rührung in einem geschlossenen Reaktionsgefäß auf 40 - 60° C, aber nicht über 140° C, vorzugsweise auf 70 - 90° C, erhitzt. Der Reaktionsablauf wurde mit Hilfe des Brechungsindex, der ein direktes Maß für den Umsetzungsgrad des Diisocyanatgemisches bzw. gegebenenfalls des reinem Diisocyanats ist, bestimmt. Bei einem Umsatz von ca. 30 - 45 %, bezogen auf das eingesetzte Diisocyanatgemisch, wurde die Reaktion durch Kühlung des Reaktionsgemisches auf Raumtemperatur abgebrochen. Bei Raumtemperatur waren solche Diisocyanat/Isocyanurat-Gemische lagerbeständig. Zur Abtrennung des nicht umgesetzten, d.h. des monomeren Diisocyanats bzw. Diisocyanatgemisches und des Katalysators wurde das Reaktionsgemisch dann der Dünnschichtverdampfung (150° C/0,1 Torr) zugeführt. Die so hergestellten erfindungsgemäß Isocyanurate hatten einen NCO-Gehalt von 19 - 24, vorzugsweise 20,5 - 22,5 Gew.%, einen Monomerengehalt $<0{,}7$ Gew.% und eine Viskosität unter 12.000 mPas bei 25° C.

Die erfindungsgemäßen Isocyanat-isocyanurate besitzen; eine Reihe vorteilhafter Eigenschaften, die sie als Isocyanathärter für Beschichtungssysteme nach dem; Isocyanat-Polyadditionsverfahren hervorragend geeignet machen. Aufgrund ihrer niedrigen Viskosität, die in Abhängigkeit vom Oligomerisationsgrad schwankt, jedoch im allgemeinen unter 12.000 mPas (bei 25° C) liegt, werden für ihren Einsatz keine oder nur geringe Mengen an Lösungsmitteln benötigt.

Der wesentliche Vorteil gegenüber Polyisocyanaten nach dem Stand der Technik liegt in der besseren Verträglichkeit der erfindungsgemäßen Verbindungen mit unpolaren Lösungsmitteln, die die Anwendungsbreite dieser gegenüber herkömmlichen Produkten wesentlich vergrößert. Außerdem ist die überraschend gute Thermo-wie Oxidationsbeständigkeit der erfindungsgemäßen Verbindungen hervorzuheben.

## A. Herstellung der als Katalysatoren eingesetzten Ammoniumsalze

### Beispiel A1

In einem mit Rührer, Rückflußkühler und Tropftrichter versehenen 1 l-Dreihalskolben wurden 232 g Dipropylenglykol (DPG) und 90 g Eisessig gegeben. In diese Mischung wurde dann bis zu einer Gewichtszunahme von 87 g Trimethylamin eingeleitet. Anschließend wurde langsam bei 25° C unter intensiver Rührung 87 g Propylenoxid (PO)zugegeben. Nach Beendigung der PO-Zugabe wurde die Mischung bei Raumtemperatur über Nacht gerührt, dann im Vakuum innerhalb von 6 h bei 45° C die nicht umgesetzten flüchtigen Materialien abgezogen. Es blieb ein Rückstand mit einem Gewicht von 484 g zurück.

Unter Anwendung der oben beschriebenen Verfahrensweise wurden weitere Verbindungen hergestellt, deren Zusammensetzung in der folgenden Tabelle 1-(Beispiel A2 - A6) zusammengestellt ist.

Tabelle: Trimesierungskatalysatorer

| Beispiel | Amin | Säure | Alkylenoxid | Lösungsmittel |
|---|---|---|---|---|
| A2 | Trimethylamin | Essigsäure | Propylenoxid | Dipropylenglykol |
| A3 | Trimethylamin | Ameisensäure | Propylenoxid | Dipropylenglykol |
| A4 | N-N'-Endoethylen-piperazin | Ameisensäure | Propylenoxid | keine |
| A5 | N-Methylmorpholin | Cyanessigsäure | Propylenoxid | Dipropylenglykol |
| A6 | Trimethylamin | 2-Ethylhexansäure | Propylenoxid | Dipropylenglykol |

**Beispiel A7**

500 Gew.Teile Triethylendiamin wurden mit 500 Gew.-Teilen Propylenoxid 8 h unter Rückfluß erhitzt. Mit zunehmender Reaktionsdauer wurde das Reaktionsgemisch immer dunkler. Nach beendeter Reaktion veränderte sich die Leitfähigkeit dieses Gemisches nicht mehr.

**Beispiel A8**

500 Gew.Teile Triethylendiamin wurden in 500 Gew.-Teilen Dimethylformamid gelöst. Zu dieser Lösung wurden dann bei Raumtemperatur 500 Gew.Teile Propylenoxid zugegeben, anschließend wurde diese Lösung 18 h bei 40°C erhitzt. Danach war die Reaktion beendet. Die Leitfähigkeit dieser Lösung veränderte sich beim Lager nur noch unwesentlich.

**B. Herstellung der erfindungsgemäßen Isocyanatoisocyanurate**

**Beispiel B1**

500 Gew.Teile MPDI (ca. 6 % EBDI) und 0,2 Gew. Teile des Katalysators des Beispiels A6 wurden unter intensiver Rührung miteinander bei 80°C gemischt. Dabei trat sofort eine Wärmetönung auf, wobei die Temperatur des Reaktionsgemisches auf ca. 95°C stieg. Bei dieser Temperatur wurde das Reaktionsgemisch noch 20 Minuten weiter erhitzt. Während dieser Zeit fiel der NCO-Gehalt auf 35,4 Gew.%. Zur Entfernung des nicht umgesetzten MPDI wurde das Reaktionsgemisch bei 160°C/0,1 Torr in einem Dünnschichtverdampfer destilliert. Das Reaktionsprodukt (Rückstand der Dünnschichtverdampfung) hatte einen NCO-Gehalt von 20,5 Gew.% und einen Monomerengehalt von <0,7 Gew.%. Die Viskosität betrug bei 25°C 11.500 mPas.

**Beispiel B2**

Entsprechend dem in Beispiel B1 beschriebenen Verfahren wurden 1.000 Gew.Teile MPDI und 0,1 Gew.-Teile des in Beispiel A7 beschriebenen Katalysators bei 80°C zur Reaktion gebracht. Nach Beendigung der partiellen Trimerisierung hatte das Reaktionsgemisch einen NCO-Gehalt von 38,2 Gew.%. Das durch Dünnschichtdestillation isolierte Isocyanato-isocyanurat hatte einen NCO-Gehalt von 21,3 Gew.% und einen Monomergehalt von 0,6 Gew.%. Die Viskosität dieses Isocyanato-isocyanurats betrug bei 25°C 10.900 mPas.

**Beispiel B3**

In analoger Weise zu Beispiel B1 wurden 1.000 Gew.-Teile MPDI und 0,15 Gew.Teile des in A1 beschriebenen Katalysators bei 80°C zur Reaktion gebracht. Nach beendeter partieller Trimerisierung hatte das Reaktionsgemisch einen NCO-Gehalt von 41,1 Gew.%. Das durch Dünnschichtdestillation isolierte Isocyanato-isocyanurat hatte einen NCO-Gehalt von 25,7 Gew.% und einen Monomergehalt von 0,7 Gew.%. Die Viskosität dieses Isocyanato-isocyanurats betrug bei 25°C 10.500 mPas.

**Patentansprüche**

1. Isocyanato-isocyanurate der Formel

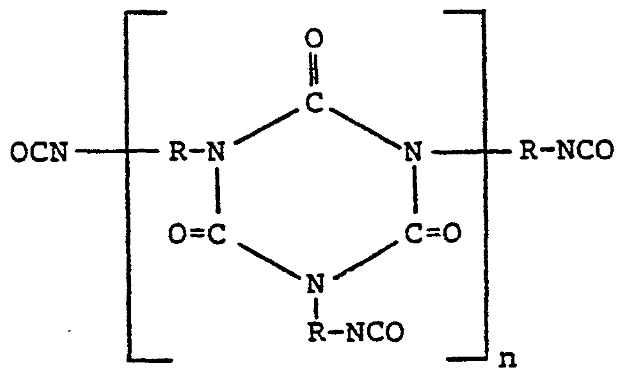

in welcher die einzelnen Reste R gleich oder verschieden sind und Kohlenwasserstoffreste der Formeln

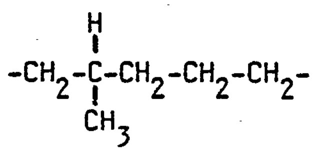 oder 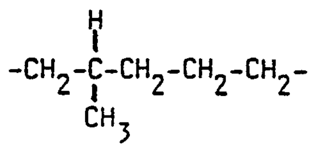 und 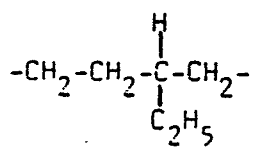

bedeuten und n eine ganze oder gebrochene Zahl von 1 - 5 bedeutet.

2. Verfahren zur Herstellung von Isocyanato-isocyanuraten gemäß Anspruch 1 durch katalytische Trimerisierung eines Teils der Isocyanat-Gruppen von aliphatischen Diisocyanaten,
dadurch gekennzeichnet,
daß man als aliphatische Diisocyanate ein im wesentlichen aus einem Diisocyanat der Formel

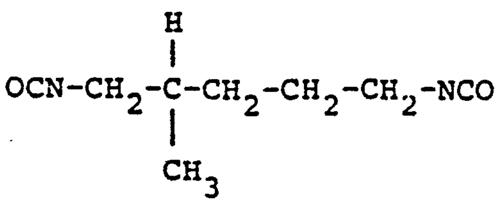

und gegebenenfalls einem Diisocyanat der Formel

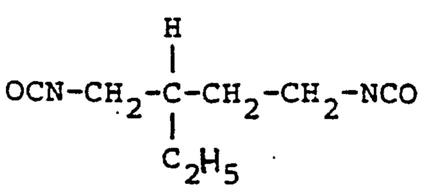

bestehendes Diisocyanat bzw. Diisocyanat-Gemisch verwendet,
wobei der Mindestgehalt an 2-Ethyl-1,4-diisocyanato-butan 1 Gew.-% und die Menge an Katalysator 0,2-0,02 Gew.-% beträgt und der Temperaturbereich bei 40 - 140 °C liegt.

**Claims**

1. Isocyanato-isocyanurates of the formula

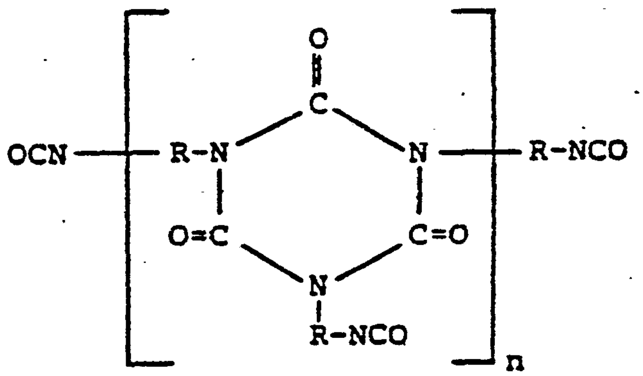

in which the individual radicals R are identical or different and denote hydrocarbon radicals of the formulae

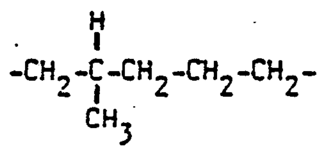 or 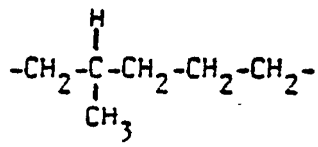 and 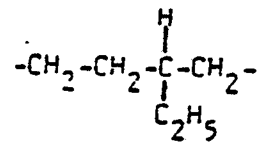

and n denotes an integral or fractional number between 1 and 5.

2. Process for the preparation of isocyanatoisocyanurates according to Claim 1 by catalytic trimerization of a part of the isocyanate groups of aliphatic diisocyanates, characterized in that as aliphatic diisocyanates there is used a diisocyanate, or diisocyanate mixture, essentially consisting of a diisocyanate of the formula

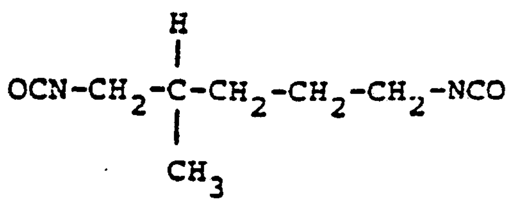

and, optionally, a diisocyanate of the formula

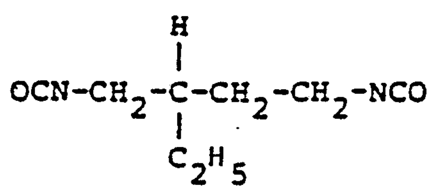

the minimum content of 2-ethyl-1,4-diisocyanatobutane being 1% by weight and the amount of catalyst being 0.2 - 0.02% by weight and the temperature range being 40 - 140°C.

## Revendications

1°) Ioscyanato-isocyanurates répondant à la formule

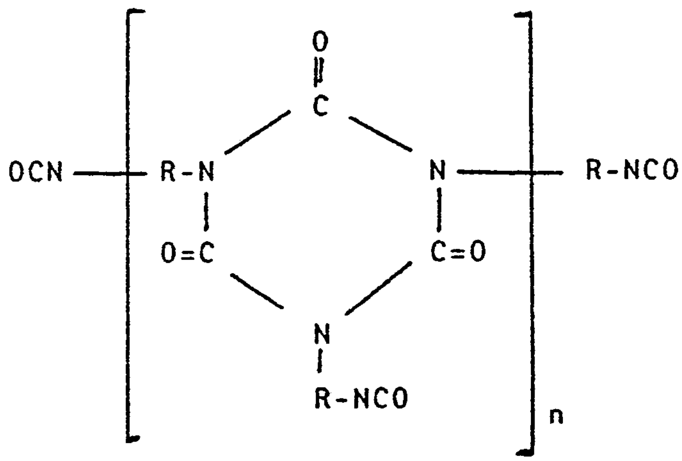

où les différents restes R sont semblables ou différents, et représentent des restes hydrocarbures répondant aux formules:

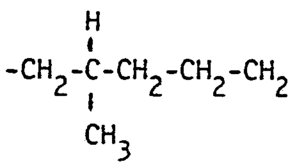 ou 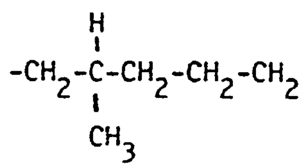 et 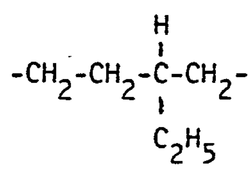

et n est un nombre entier ou fractionnel de 1 à 5.

2°) Procédé pour la fabrication d'isocyanato-isocyanurates suivant la revendication 1, par trimérisation catalytique d'une partie des groupes isocyanates de diisocyanates aliphatiques, caractérisé en ce que comme diisocyanate aliphatique, on utilise essentiellement un diisocyanate répondant à la formule :

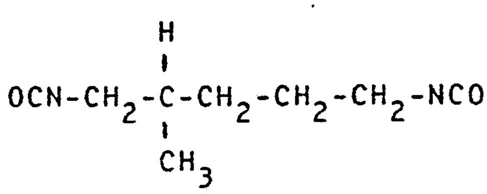

et éventuellement un diisocyanate répondant à la formule:

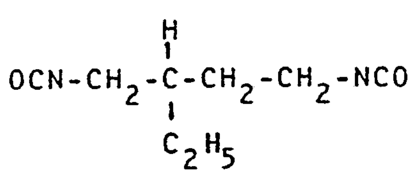

ou un mélange des isocyanates ci-dessus, la teneur minimum en 2-éthyl-1,4-diisocyanatobutane étant de 1% en poids, la teneur en catalyseur se montant à 0,2 à 0,02 % en poids et la plage de température se situant de 40 à 140°C.